# EUROPEAN PATENT APPLICATION

(11) **EP 2 022 464 A2**
(43) Date of publication of application: **11.02.2009**
(21) Application number: 08252590.8
(22) Date of filing: 30.07.2008
(51) Int. Cl.: A61K 6/10, A61C 9/00

(54) **Gingival retraction with light curable composition**

(30) Priority: 07.08.2007 US 835139
(71) Applicant: Kerr Corporation, Orange, CA 92867 (US)
(72) Inventor: Chen, Xiangxu, Diamond Bar, CA 91765 (US); Qian, Xuejun, Foothill Ranch, CA 92610 (US)
(74) Representative: Findlay, Alice Rosemary

(57) **Abstract**

A method for temporarily widening a gingival sulcus. A composition comprising a polymerizable monomer having at least one ethylenically unsaturated group, a photo polymerization initiator, and a fine inorganic powder is inserted within a gingival sulcus to be widened, and the composition is thereafter irradiated in situ to polymerize the composition. The result is a rubbery material that is easily removable from the widened sulcus.

## Description

The gingiva is the soft mucosal tissue that connects teeth and bone. It is a common practice for dental practitioners to retract (i.e., temporarily widen) a gingival sulcus for further dental treatments, such as impressions.

Methods to retract a gingival sulcus may be classified as mechanical, chemo-mechanical, rotary curettage, and electro-surgical methods.

Mechanical methods involve placing a string into the gingival sulcus to physically displace the tissue. Gingival retraction cords are commercially available, e.g., Ultrapak^{™} (Ultradent, South Jordan UT). During gingival retraction procedures, gingival reaction cords are packed and maintained between the gingiva and tooth, then are removed before further dental treatments. Dental practitioners generally find retraction cord packing a time-consuming and frustrating procedure. Bleeding and oozing may also result from pressure applied during the procedure.

Chemo-mechanical methods involve treatment with one or more chemicals that may shrink the tissues temporarily and may also control hemorrhage. An astringent agent, also referred to as an astrigent, is such a chemical; it shrinks or constricts body tissues. This effect is usually local after topical application. Astringents have been used in gingival retraction procedures to stop bleeding or oozing. Chemicals commonly used as astringents in the chemo-mechanical method may be alums (sulfates that have the typical formula M⁺₂SO₄·M³⁺₂(SO₄)₃·24H₂O, where M⁺ denotes the sign of an alkali metal or ammonium ion and M³⁺ denotes one of the trivalent metal ions, typically aluminum, chromium, or iron (III)); aluminum chloride; aluminum sulfate; ferric chloride; ferric sulfate; zinc chloride; zinc sulfate; and/or epinephrine. Aluminum chloride, ferric sulfate, and epinephrine are the most widely used astringents.

Dental tools have been developed to facilitate gingival retraction and may be used alone or with other treatments. For example, lasers can promote gum healing, reattach gum tissues to root surfaces, and destroy bacteria involved in gum diseases.

Such procedures are time-consuming and require skills in application and use, and are exacerbated when gingival retractions are applied on several teeth at the same time.

While cordless mechanochemical gingival retraction materials have been developed, and while astringent chemicals may be included to effectively cause tissue or blood vessel to contract to further control oozing of gingival tissue, other compositions and methods are desirable.

The invention will now be described by way of the following detailed description of preferred embodiments.

One embodiment of the invention is a gingival retraction composition that can be polymerized by light irradiation after gingival placement and can be easily removed after use. One embodiment is a method for widening a gingival sulcus, which is the crevice that surrounds a tooth, by injecting the composition subsequently described into the gingival sulcus and then polymerizing the composition by light irradiation to form a material that is easily removed with instruments. The composition is elastomeric (i.e., rubbery).

This composition contains (a) a polymerizable monomer having at least one ethylenically unsaturated group; (b) a photo polymerization initiator; and (c) a fine inorganic powder.

Component (a) is a radically polymerizable monomer(s) having one or more ethylenically unsaturated group. The ethylenically unsaturated group may be (meth)acrylate (= acrylate or methacrylate), vinyl, (meth)acrylamide (= acrylamide or methacrylamide) groups. Mono-functional monomers include, but are not limited to, methyl (meth)acrylate, ethyl (meth)acrylate, propyl (meth)acrylate, butyl (meth)acrylate, hydroxyethyl (meth)acrylate, hydroxypropyl (meth)acrylate, glycerol mono(meth)acrylate, polyethyleneglycol mono-(meth)acrylate, polypropyleneglycol mono-(meth)acrylate, polytetramethyleneglycol mono-(meth)acrylate, (meth)acylamide, N-methyl(meth)acrylamide, N-ethyl(meth)acrylamide, N-butyl(meth)acrylamide, N,N-dimethyl(meth)acrylamide, N,N-diethyl(meth)acrylamide, N,N-dibutyl(meth)acrylamide, (3-acryloylaminopropyl)trimethylammonium chloride, [3-(methacryloylamino) propyl] trimethylammonium chloride, and/or [3-(methacryloylamino)propyl]dimethyl(3-sulfopropyl)ammonium hydroxide. Multifunctional monomers include, but are not limited to, glycerol di(meth)acrylate, glycerol tri(meth)acrylate, 2,2-bis[4-(2-hydroxy-3-methacryloylpropoxy)-phenyl]-propane (bisGMA), urethane di(meth)acrylate, ethoxylated bisphenol A dimethacrylate (EBPADMA-n where n=total number of moles of ethylene oxide in the molecule, as only one example, n = 2-50 units), ethylene glycol di(meth)acrylate, neopentyl glycol di(meth)acrylate, tetraethylene glycol di(meth)acrylate, triethylene glycol di(meth)acrylate, diethylene glycol di(meth)acrylate, 1,3-butylene glycol di(meth)acrylate, cyclohexane dimethanol di(meth)acrylate, 1,6-hexanediol di(meth)acrylate, 1,4-butanediol dimethacrylate, propoxylated glyceryl tri(meth)acrylate, polyethyleneglycol di-(meth)acrylate, polypropyleneglycol di-(meth)acrylate, polytetramethyleneglycol di-(meth)acrylate, hexanediol di(meth)acrylate, trimethylolpropane tri(meth)acrylate, ethoxylated trimethylolpropane tri(meth)acrylate, tris (2-hydroxy ethyl) isocyanurate tri(meth)acrylate, pentaerythritol di(meth)acrylate, pentaerythritol tri(meth)acrylate, pentaerythritol tetra(meth)acrylate, dipentaerythritol penta(meth)acrylate, multifunctional aliphatic urethane (meth)acrylate, multifunctional aromatic urethane (meth)acrylate, N,N'-methylenebis-(acrylamide), N,N'-ethylenebis(acrylamide), and/or N,N'-butylenebis(acrylamide).

In one embodiment, the ethylenically unsaturated monomers have flexible units and are rubbery after curing; such a material is also referred to as an elastomer. Examples of monomers that can form a rubbery material after curing include, but are not limited to, polyethyleneglycol (PEG) mono- or di- (meth)acrylate with the molecular weight of PEG ranging from 400 to 5000, polypropyleneglycol (PPG) mono- or di- (meth)acrylate with the molecular weight of PPG ranging from 400 to 5000, polyisopropyleneglycol mono- or di-(meth)acrylate with the molecular weight of polyisopropyleneglycol ranging from 400 to 5000, polytetramethyleneglycol mono- or di- (meth)acrylate with the molecular weight of polytetramethyleneglycol ranging from 400 to 5000, EBPADMA-n (where n is greater than 20), ethoxylated trimethylolpropane tri(meth)acrylate, elastomeric urethane (meth)acrylate oligomers that contain PEG or PPG segments with an average molecular weight of 500 to 5000.

In one embodiment, the monomers have a solubility in water of more than 1 wt %. In one embodiment, the monomers have a solubility in water of more than 5 wt %. In one embodiment, the monomers have a solubility in water of more than 15 wt %. Examples of such monomers include, but are not limited to, EBPADMA-n (where n is greater than 20), ethoxylated trimethylolpropane tri(meth)acrylate, polyethyleneglycol di-(meth)acrylate, multifunctional aliphatic urethane (meth)acrylate, N,N-diethyl(meth)acrylamide and (3-Acrylamidopropyl)trimethylammonium chloride.

In one embodiment, the concentration of ethylenically unsaturated monomer(s) in the total composition ranges from about 0.05% by weight to about 80% by weight inclusive.

Component (b) is a photo polymerization initiator, also referred to as a photoinitiator, that initiates polymerization of the composition. In one embodiment, a dental curing light capable of generating ultraviolet (UV) and/or visible light is used. In one embodiment, the concentration of the initiator in the total composition ranges from about 0.001% by weight to about 5% by weight inclusive.

In one embodiment, the photoinitiator is a photosensitizer and a reducing agent. Photo-initiators/sensitizers include, but are not limited to, camphorquinone (CQ), phenathrenequinone, 4,4'-bis(dimethylamino)benzophenone, and/or 4,4'-bis(diethylamino)benzophenone. CQ absorbs both ultraviolet light and visible light. Amines, including but not limited to tertiary amines, can be used as reducing agents for CQ to co-initiate free radical polymerization. Examples of tertiary amines include, but are not limited to, ethyl-4-(N,N-dimethylamino) benzoate (EDMAB), 2-ethylhexyl-4-(N,N-dimethylamino) benzoate (ODMAB), 4-dimethylamino-benzophenone (DMABP), p-dimethylamino benzoic acid (DMABA), p-(dimetnylamino) benzonitrile (DMABCN), p-(dimetnylamino) benzaldehyde, 4'-morpholino-acetophenone, 4'-morpholino-benzophenone, p-(dimethylamino) acetophenone, 4,4'-bis(dimethylamino)-benzophenone, 4,4'-bis(diethylamino) benzophenone, and/or dimethylaniline. In one embodiment, the tertiary amines EDMAB, ODMAB, DMABP, DMABA and/or DMABCN are used. Other reducing agents for CQ include, but are not limited to, chemical compounds with urethane and benzhydyl groups.

In one embodiment, the photoinitiator is a phosphine oxide, which include mono-acyl and multi-acyl phosphine oxide. Phosphine oxides can initiate free radical polymerizations by themselves under UV and/or visible irradiation generated by a typical dental curing device. Examples of phosphine oxides include, but are not limited to, bis(2,4,6-trimethylbenzoyl)-phenyl phosphine oxide (Irgacure 819, Ciba Specialty Chemicals, Basel Switzerland), bis(2,6-dimethoxybenzoyl)-(2,4,4-trimethylpentyl) phosphine oxide (CGI 403, Ciba Specialty Chemicals), and/or ethyl 2,4,6-trimethylbenzoyl-phenyl phosphine oxide (LUCIRIN LR8893X, BASF Corp., Charlotte NC). In one embodiment, two or more phosphine oxides may be combined. An example of combinations of phosphine oxides includes, but is not limited to, a 50:50 by weight mixture of 2,4,6-trimethylbenzoyl-diphenyl phosphine oxide and 2-hydroxy-2-methyl-1-phenylpropane-1-one (DAROCUR 4265, Ciba Specialty Chemicals).

In one embodiment, fluoron and pyronin derivatives initiate free radical polymerization, together with amines and iodonium synergists under UV and/or visible irradiation generated by a typical dental curing device. An example of a fluoron derivative includes, but is not limited to, 5,7-diiodo-3-butoxy-6-fluorone (H-Nu 470, Spectra Group Ltd., Millbury OH). Other examples of fluoron and pyronin derivatives that can initiate free radical polymerizations are described in U.S. Patent Nos. 5,623,080 to Neckers and Shi, and 5,451,343 to Neckers and Shi, each of which is expressly incorporated by reference herein.

Component (c) is a fine inorganic powder is incorporated into the composition. A fine powder is one in which the mean particle size is less than about 50 microns. In one embodiment, the inorganic powder is a thickening agent that significantly increases the viscosity of the composition. Examples of fine inorganic powders include, but are not limited to, silicas and clays. Both micrometer size and nanometer size powders can be used in compositions. In one embodiment, the mean particle size is less than about 20 microns. In one embodiment, the mean particle size is less than about 10 microns.

Examples of silicas include, but are not limited to, fumed silica, colloidal silica, and/or precipitated silica. Examples of colloidal and fumed silicas include, but are not limited to, Aerosil series and AERODISP series (both from Degussa, Ridgefield Park NJ), and Cab-O-Sil series (Cabot Corp., Tuscola IL). Aerosil series include, but are not limited to, Aerosil 150, 200, 300, 380, R202, R805, R972, OX-50, OX-130 and OX200 silica. AERODISP series include, but are not limited to, AERODISP W1714, W1824, W1836, W630, W7512S and W7520, all of which are water-based dispersions. Cab-O-Sil series include, but are not limited to, Cab-O-Sil M5, LM-150, TS-720, TS-610, and TS-530. The thickening agent also includes nanoparticles such as those obtained through a sol-gel process. Examples include those disclosed in U.S. Patent Nos. 4,567,030 to Yuasa et al. and 5,609,675 to Noritake and Yuasa, each of which is expressly incorporated by reference herein. The surface of a silica may be treated or coated with a coupling agent, such as gamma-methacryloyloxypropyltrimethoxy-silane (MPTMS). In one embodiment, silica has an average particles size of less than 1 micrometer. In another embodiment, silica has an average particle size of 100 nanometers.

Clays are naturally occurring fine-grain particles in sediment, soil, or rock. Clays contain a variety of phyllosilicate minerals rich in silicon, aluminum oxides, hydroxides, and a variety of structural water. Clays are distinguished from other small particles present in sediment/soil/rock, such as silt and sand, by their small size, flake or layered shape, affinity for water, and high plasticity. Clays may have high plasticity when mixed with certain amounts of water. Clays include the following groups: kaolinite, smectite, illite, and chlorite. Kaolinites include the minerals kaolinite, dickite, halloysite, and ancrite. Smectites include pyrophyllite, talc, vermiculite, sauconite, saponite, nontronite, and montmorillonite. Illites include micas. Chlorites include a variety of similar minerals with considerable chemical variation. Clays of kaolinite and smectite groups are used for skin care applications. Montmorillonite is a very soft mineral of the smectite group. It has two tetrahedral sheets sandwiching a central octahedral sheet, also known as a 2:1 clay. Kaolinite has one tetrahedral sheet linked through oxygen atoms to one octahedral sheet of alumina octahedral, also known as a 1:1 clay. Bentonite is a clay consisting mostly of montmorillonite. Bentonite and montmorillonite are sometimes used interchangably to refer to the same mineral. Two types of bentonites exist: sodium bentonite (swelling bentonite) and calcium bentonite (non-swelling bentonite). Bentonites are formed from hydrothermal weathering of volcanic ash. The clay can be a sheet clay, which includes kaolinite, montmorillonite (bentonite), talc, mica (illite), serpentine, chlorite, mullite, kyanite, pumice, goethite, and/or pyrophyllite. In one embodiment, the clay is kaolinite and/or bentonite. In one embodiment, the clay is micronized kaolinite and bentonite.

Examples of other inorganic powders include, but are not limited to, fine particles of metals, metal oxides, metal fluorides, silicates, and aluminosilicates.

In one embodiment, the concentration of inorganic powder in the total composition ranges between about 0.1 % by weight to about 90 % by weight inclusive. In one embodiment, mixtures of different inorganic powders can be used.

In one embodiment, the composition may further contain a solvent. The solvent dissolves or disperses monomers, initiators, and other ingredients. The solvent also wets fillers. Both protic and aprotic solvents may be used. A protic solvent is any solvent that carries hydrogen attached to oxygen or nitrogen. Examples of hydrogen attached to oxygen include, but are not limited to, hydroxyl, carboxylic acid, and phosphoric acid groups. An examples of hydrogen attached to nitrogen includes, but is not limited to, an amine group. Protic solvents include, but are not limited to, water, ethyl alcohol, propyl alcohol, isopropyl alcohol, butyl alcohol, *tert*-butyl alcohol, glycerin, polyethylene glycol, polypropylene glycol, pentaerythritol ethoxylate, acetic acid, and/or fatty acids. In one embodiment, the concentration of protic solvent(s) in the composition ranges from about 0.1 % by weight to about 60 % by weight inclusive. An aprotic solvent is any solvent that does not carry hydrogen attached to oxygen or nitrogen. Aprotic solvents include, but are not limited to, acetone, methyl ethyl ketone, ethyl acetate, tetrahydrofuran, and diethyl ether. In one embodiment, the concentration of aprotic solvent(s) in the composition is in the range of about 0.1 % by weight to about 50 % by weight inclusive. In one embodiment, more than one solvent is used.

In one embodiment, the composition further contains an astringent. Astringents include, but are not limited to, alums, aluminum chloride, aluminum sulfate, ferric chloride, ferric sulfate, zinc chloride, zinc sulfate, and/or epinephrine. In one embodiment, astringents incorporated in the composition include aluminum chloride, ferric sulfate, and/or epinephrine. In one embodiment, the total concentration of astringent in the composition ranges from about 0.1 % by weight to about 30 % by weight inclusive. In one embodiment, the total concentration of astringent in the composition ranges from about 10 % by weight to about 20 % by weight, inclusive.

In one embodiment, the viscosity of the composition is measured by a dynamic stress rheometer and is higher than about 13000 Pascals·second. In one embodiment, the viscosity of the composition is measured using a penetrometer, or by other methods known to one skilled in the art. In one embodiment, a universal penetrometer is used to measure viscosities of a wide variety of materials using penetration of weighted needles. A plunger is released to penetrate into viscous pastes, and depth of penetrations are used to compare viscosities. A Precision 73515 (Houston TX) universal penetrometer is employed to evaluate paste viscosities using American Society for Testing and Materials (ASTM) D-5. The total weight of the plunger rod and the penetrating needle is 50 grams and extra weight may be added to bring the total weight of penetration to between 100 grams and 150 grams. The diameter of the penetrating needle is 1 mm. The duration of penetrations is set to be ten seconds. The sample container has a diameter of 10 mm and a depth of 8 mm. Three penetrations may be applied on each freshly prepared sample at 24°C ± 1°C. Using the penetrometer, the probe without additional weight penetrated about 1.1 mm on EXPASYL® (Kerr, Orange CA). In one embodiment, the penetration depth without additional load on the disclosed composition was between about 0.01 mm to about 7.5 mm. In one embodiment, the penetration depth was between about 0.05 mm to about 3 mm. In one embodiment, the penetration depth was between about 0.1 mm to about 2 mm.

In one embodiment, a pH buffering agent may be included to make the composition less acidic and hence more biocompatible. Buffering agents include, but are not limited to, sodium bicarbonate, sodium carbonate, potassium bicarbonate, and/or potassium carbonate. In one embodiment, the total concentration of pH buffering agent in the composition ranges between about 0.01 % by weight to about 10 % by weight. In one embodiment, the total concentration of pH buffering agent in the composition ranges between is between about 0.1 % by weight to about 5 % by weight.

In one embodiment, a flavorant and/or odorant may be included to impart a more desirable taste and/or smell to the composition. These include, but are not limited to, citrus (e.g., orange, lime), mint (e.g., peppermint), isoamyl acetate, ethyl propionate, and/or ethyl maltol. In one embodiment where a flavorant is included, the concentration of flavorant ranges between about 0.0001 % by weight to about 5 % by weight inclusive. In one embodiment where a flavorant is included, the concentration of flavorant ranges between about 0.001 % by weight to about 2 % by weight inclusive.

In one embodiment, a colorant may be included to introduce a particular and/or distinctive color to the composition. Colorants include, but are not limited to, dyes, pigments, and inks. In one embodiment, food dyes are used which include, but are not limited to, Brilliant Blue FCF, indigotine, Fast Green FCF, Allura Red AC, tartrazine, and/or Orange Yellow S. In one embodiment where a colorant is used, the concentration of colorant in the composition is between about 0.0001 % by weight to about 3 % by weight inclusive. In one embodiment where a colorant is used, the concentration of colorant is between about 0.001 % by weight to about 1 % by weight inclusive.

A radiopaque agent with enhanced x-ray absorbing power can be incorporated to increase the radiopacity of the composition. An increased radiopacitiy permits easy detection with X-ray. In one embodiment, the radiopaque agent is an inorganic filler with increased X-ray contrast ability. Such inorganic fillers include, but are not limiting to, metals, salts, oxides, fluorides, silicate glass, aluminosilicate glass, aluminoborosilicate glass, and/or fluoroaluminosilicate glass containing elements of high atomic number such as Sr, Y, Zr, Ba, La, Hf, Zn, Bi, W, rare earth metals. Examples include, but not limited to, barium sulfate, silver, strontium fluoride, barium fluoride, ytterbium fluoride, yttrium fluoride, barium tungstate, zinc oxide, bismuth(III) oxide, bariumaluminosilicate, bariumaluminoborosilicate, strontiumaluminosilicate, bariumfluoroaluminosilicate, strontiumfluoroaluminosilicate, strontiumzincfluoroalumino-silicate, and/or zincaluminosilicate. In one embodiment, the fine inorganic powder in the composition functions as a thickening agent as well as a radiopaque agent. In another embodiment, the radiopaque agent is added in addition to the thickening agent.

In one embodiment, a stabilizer is added to obtain a chemically stable composition that has a desirable shelf-life. Stabilizers include, but are not limited to, 3,5-di-tert-butyl-4-hydroxytoluene (BHT) and hydroquinone monomethyl ether (MEHQ). In one embodiment, the concentration of the stabilizer is between about 0.0001 % by weight to about 5% by weight inclusive of the composition.

The composition may be inserted into the gingival sulcus by various methods that include, but are not limited to, an injection device. In one embodiment, the composition is injected into the gingival sulcus using a device having a needle with a diameter between about 0.2 mm to about 2 mm that contacts gingival tissue. In one embodiment, the composition is injected into the gingival sulcus using a device having a needle with a diameter between about 0.7 mm to about 1.6 mm that contacts gingival tissue. Other diameter needles may be determined by actual applications. In one embodiment, the composition remains in the sulcus for between about one second to about fifteen minutes. In one embodiment, the composition remains in the sulcus for between about ten seconds to about five minutes. Due to the high viscosity of the composition, in one embodiment the gingival sulcus is widened to obtain a retraction effect. Multiple injections may be needed to achieve desired retraction. In one embodiment, bleeding of gingival tissue is controlled by an astringent agent.

After the gingival tissue is effectively widened, the composition is then irradiated by a curing light. Curing lights include, but are not limited to, a halogen light and LED (light-emitting diode) light. In one embodiment, the range of curing time is between about one second to about 300 seconds. Polymerization occurs to form a one-piece composition that can be manually removed from the gingival sulcus. In one embodiment, the polymerized composition is a rubber that can be extended to an extra length of about 0.5% to about 300%. In one embodiment, a dental instrument is used to remove the polymerized composition.

The following examples illustrate embodiments and uses of the composition, and do not limit the scope of the disclosure.

In the examples the following materials were used:
CQ: camphorquinone
EDMAB: ethyl-4-(N,N-dimethylamino) benzoate
OX-50: fumed silica or colloidal silica sold by Degussa
TS-530: surface treated fumed silica or colloidal silica sold by Cabot Corp
SR9036A: a ethoxylated(30) bisphenyl A dimethacrylate sold by Sartomer (Exton PA)
Blue 2: Blue 2 sold by Warner-Jenkinson Co. (St Louis MO)
BR7432G: High elongation urethane acrylate by Bomar (Winsted CT)

**Example 1**

| | |
|---|---|
| OX-50 | 22.0% |
| TS-530 | 22.0% |
| SR9036A | 55.0% |
| CQ | 0.39% |
| EDMAB | 0.55% |

Using a penetrometer, a probe without additional weight penetrated about 1.3 mm. Upon irradiation with a dental halogen curing light (Optilux 501, 500 mW/cm³) for 20 seconds, the composition became a rubbery material.

**Example 2**

| | |
|---|---|
| OX-50 | 22.0% |
| TS-530 | 22.0% |
| SR9036A | 55.0% |
| Blue 2 | 0.03% |
| CQ | 0.39% |
| EDMAB | 0.55% |

Using a penetrometer, the probe without additional weight penetrated about 2.1 mm. Upon irradiation with a dental halogen curing light (Optilux 501, 500 mW/cm³) for 20 seconds, the composition became a rubbery material.

**Example 3**

| | |
|---|---|
| SR9036A | 44.1% |
| Water | 11.0% |
| Bentonite | 44.1% |
| CQ | 0.33% |
| EDMAB | 0.44% |

Using a penetrometer, the probe without additional weight penetrated about 3.1 mm. Upon irradiation with a dental halogen curing light (Optilux 501, 500 mW/cm³) for 20 seconds, the composition became a rubbery material.

**Example 4**

| | |
|---|---|
| | |
| SR9036A | 34.6% |
| Water | 13.0% |
| Bentonite | 51.9% |
| CQ | 0.26% |
| EDMAB | 0.35% |

Using a penetrometer, the probe without additional weight penetrated about 0.3 mm. Upon irradiation with a dental halogen curing light (Optilux 501, 500 mW/cm³) for 20 seconds, the composition became a rubbery material.

**Example 5**

| | |
|---|---|
| SR9036A | 37.8% |
| Water | 14.2% |
| Bentonite | 47.3% |
| CQ | 0.28% |
| EDMAB | 0.38% |

Using a penetrometer, the probe without additional weight penetrated about 1.1 mm. Upon irradiation with a dental halogen curing light (Optilux 501, 500 mW/cm³) for 20 seconds, the composition became a rubbery material.

**Example 6**

| | |
|---|---|
| Aluminum chloride | 11% |
| Water | 14% |
| Borosilicate glass filler | 34.6% |
| Bentonite | 14.7% |
| BR7432G | 25.7% |
| CQ | 0.15% |
| EDMAB | 0.37% |

Using a penetrometer, the probe without additional weight penetrated about 1.7 mm. Upon irradiation with a dental halogen curing light (Optilux 501, 500 mW/cm³) for 20 seconds, the composition became a rubbery material.

The disclosure is not limited to the specific embodiments described and exemplified, and other embodiments will be appreciated by one skilled in the art.

## Claims

1. Use of a composition for temporarily widening a gingival sulcus, the use comprising inserting the composition within a gingival sulcus to be widened, and thereafter photo curing to polymerize the composition, the composition comprising a polymerizable monomer having at least one ethylenically unsaturated group, a photo polymerization initiator, and a fine inorganic powder, the method temporarily widening the gingival sulcus.

2. Use of claim 1 wherein the composition remains in the sulcus from about one second to about fifteen minutes before irradiating.

3. Use of either claim 1 or claim 2 wherein the composition is inserted into the gingival sulcus through a device with a needle.

4. Use of any preceding claim wherein the composition further comprises a solvent selected from the group consisting of protic solvents, aprotic solvents, and combinations thereof.

5. Use of any preceding claim wherein the ethylenically unsaturated group is selected from the group consisting of acrylate, methacrylate, vinyl, acrylamide, methacrylamide, and combinations thereof.

6. Use of any preceding claim wherein the concentration of polymerizable monomer having at least one ethylenically unsaturated group ranges between 0.05% by weight to 80% by weight inclusive of the compostition, the concentration of the photo polymerization initiator in the composition ranges from about 0.001 % by weight to about 5% by weight inclusive, and the concentration of the inorganic powder ranges from about 0.1 % by weight to about 90% by weight inclusive of the composition.

7. Use of any preceding claim wherein the polymerizable monomer is substantially soluble in water.

8. Use of any preceding claim wherein the polymerizable monomer yields an elastomeric material upon polymerization.

9. Use of any preceding claim wherein the photo polymerization initiator comprises camphorquinone and a tertiary amine coinitiator.

10. Use of any preceding claim wherein the fine inorganic powder has an average particle size of less than 10 microns, preferably less than 5 microns, more preferably less than 1 micron.

11. Use of any preceding claim wherein the inorganic powder is selected from the group consisting of fumed silica, colloidal silica, precipitated silica, clay, metal oxide, metal fluoride, silicate, aluminosilicate, and combinations thereof.

12. Use of any preceding claim wherein the inorganic powder is a thickening agent that increases the viscosity of the composition.

13. Use of any preceding claim wherein the composition further comprises an astringent agent in an amount of 0.1% by weight to 30% by weight inclusive of the composition.

14. Use of claim 13 wherein the astringent agent is selected from the group consisting of alums, aluminum chloride, aluminum sulfate, ferric chloride, ferric sulfate, zinc chloride, zinc sulfate, epinephrine, and combinations thereof.

15. Use of any preceding claim wherein the composition further comprises at least one of a buffering agent, a flavorant, an odorant, and/or a colorant.

16. Use of any preceding claim wherein the inorganic powder comprises a radiopaque agent.
